# EUROPEAN PATENT SPECIFICATION

(11) **EP 1 861 051 B1**
(45) Date of publication and mention of the grant of the patent: **10.03.2010**
(21) Application number: 05817269.3
(22) Date of filing: 04.11.2005
(51) Int. Cl.: A61F 5/01

(54) **ADJUSTABLE KNEE BRACE**
VERSTELLBARE KNIESCHIENE
ATTELLE DE GENOU REGLABLE

(30) Priority: 05.11.2004 US 981851
(43) Date of publication of application: 05.12.2007
(73) Proprietor: OMNI LIFE SCIENCE, INC., Vista, CA 92081 (US)
(72) Inventor: GILMOUR, Robert, Vista, CA 92083 (US)
(74) Representative: Viering, Jentschura & Partner
(86) International application number: PCT/US2005/040043
(87) International publication number: WO 2006/052761

(56) References cited:
- US-A- 4 381 768
- US-A- 4 624 247
- US-A- 4 686 969
- US-A- 4 966 133
- US-A- 5 063 916
- US-B1- 6 436 066

## Description

### FIELD

The field of the invention is orthopedic devices, and more particularly, knee braces.

### BACKGROUND

The following includes information that may be useful in understanding the present inventions. It is not an admission that any of the information provided herein is prior art, or relevant, to the presently described or claimed inventions, or that any publication or document that is specifically or implicitly referenced is prior art.

Orthotic devices generally include a substantially rigid biomechanical element that forms the basis of the skeletal support that is required for the majority of these devices, which include braces, supports and splints.

The human knee generally comprises an articulated joint between the thigh and the lower leg muscles that supports the weight of the human body while the person is standing, walking or running. The knee joint is primarily held together by four ligaments; namely, the anterior and posterior cruciate ligaments and the medial and lateral collateral ligaments. The knee joint can be overly weakened by injuries arising out of cartilage damage and ligament strain, which may be caused, by sports injuries, as well as from everyday exercising, or physiological problems such as osteoarthritis. Thus, the human knee is subjected to a variety of stresses and strains particularly during running and jumping movements. Athletes, in particular, are apt to incur a knee injury as a result of a blow to the knee or to a twisting of the knee, which can commonly occur in various contact sports or high stress sports, such as skiing. Normal aging of the knee joint results in diminished knee stability. Muscle control may be reduced; ligaments become lax and thus less effective.

There are a variety of knee braces available on the market or through healthcare providers. These range from braces that tend to totally immobilize the knee to flexible elastic bandages that are intended to provide some flexibility while eliminating lateral movement of the ligaments that support the knee. Some of these are braces intended to be worn as a relatively permanent device for long-term wear or braces that are intended to be worn for a short period of time during overly strenuous for a short period of time, for example, for a weakened knee. The braces have as their primary object to allow for bending and straightening the knee while preventing any unnatural movement, which may aggravate the knee ligaments. While the braces are intended to allow for a natural movement of the knee joint while a person undergoes walking, running, jumping, skating, various other athletic activities, they are intended also to prevent sudden movement of the upper and lower legs to one side or the other and to prevent twisting or rotation of the lower leg relative to the upper leg about the vertical axis.

Typically, the knee braces are held in place by flexible straps, which wrap about the user's thigh and calf above and below the knee, respectively. In this manner, the rigid hinge of the knee brace remains positioned on either side of the user's knee so as to mimic the hinged joint of the knee. However, it is not uncommon for the user's bodily motions to cause the flexible straps to move relative to the person's leg, thereby misaligning the knee brace with respect to the knee. This movement of the brace straps with respect to the user not only cause misalignment and therefore misapplication of the orthotic device, but also causes irritation of the user's skin by this unintended rubbing.

Another problem with knee braces is that they must engage effectively with soft tissue in order to provide the desired support. In many parts of the body the soft tissue will move, for example by expanding or contracting as result of muscle movement. With the human leg, the thigh profile narrows a the user extends or straightens the leg from a flexed or bent position. This can cause distal migration of the knee brace with respect to the users leg, which means that the brace is not providing its desired support function. As a soft tissue changes shape, parts of the skin lose contact with the liner of the brace. This reduced contact with the liner can cause the knee brace to lose position, or move relative to the user and therefore become ineffective. The only way of overcoming this problem with existing devices is to tighten the device. This causes discomfort, prevents the skin from breathing, and can irritate the skin about the edges of the device and the liner.

The objective of any rigid knee brace is to exert a predictable force on the user's underlying skeleton. In particular, the objective is to exert a force on the tibia with respect to the femur in the user's body mass above the knee. By definition, knee braces are applied to soft tissue lying between the brace and the user's skeleton. Soft tissue is mobile and moves in a cycle corresponding to a user's gait, whether through running, walking or other physical movement common to the human knee. The most mobile soft tissue is the quadriceps mechanism lying in front of the femur in the anterior thigh region.

The central reference point for a knee brace is the knee joint line. In construction, a knee brace would use a rigid joint mechanism that mimics the movement of the knee, which is not just a simple hinge. Because each user's body shape is unique, the exact interface between the knee brace and the user's leg cannot be predetermined in the manufacture of such a device.

The function of the tibial section attached to this joint or central axis is to "fit and grip" the tibia and exert a forbe on it. In ligament instability bracing is intended to prevent anterior translocation of the tibia with respect to the femur. It is also desired to prevent the tibia deforming into varus (bow legged) or valgus (knock kneed). This is important because often injuries to the collateral ligaments coexist with cruciate ligament related pathology, and resulting instability. In bracing for osteoarthritis the objective is to exert an unloading force on the side of the knee joint most affected by degenerative pathology. This is achieved by "pushing" the tibia into a slight valgus deformity, or less frequently, a slight varus deformity.

The function of the femoral, or thigh portion, is to stabilize the central axis, or knee joint, and to provide a lever arm or counteracting force for the tibial section. Ideally the central axis, and attached tibial section, remain in a stable position relative to the actual underlying knee joint. Ideally the "lever arms" extending up the thigh remain in a stable position aligned with the underlying femur.

In US 4,624,247 a knee brace is disclosed, having a plurality of 8 flexible, inelastic, non-metallic bands connected at the plurality of points of fixation anteriorly and posteriorly of the respective braces for tightening to fit any leg and lie close to the leg. Due to the use of anterior and posterior horizontally directed fixation bands, comfort of wearing, putting on and putting off the knee brace is not very high.

What is needed is a knee brace which can more readily conform to a particular user's leg, such that the straps fit snugly, yet comfortably, about the user's leg adjacent the knee, but yet provide the adequate support so as to prevent relative movement of the knee brace with respect to the knee so that the brace provides its desired function.

There is a need for a knee brace that will overcome disadvantages of existing constructions.

### BRIEF SUMMARY

The inventions described and claimed herein have various attributes and embodiments including, but not limited to, those set forth or described or referenced in this Brief Summary.

In one aspect the invention broadly provides a knee brace having a substantially rigid support and a crossed strapping mechanism across the users thigh which adapts to thigh movement as the knee bends and straightens.

In another aspect, the invention provides a knee brace including substantially horizontal, strap.

In another aspect, the substantially horizontal strap can be adjusted to provide that the thigh elements of the brace are correctly aligned with the long axis of the femur in the mid-medial and mid-lateral lines.

In another aspect, the substantially horizontal strap can be adjusted to prevent hyperextension in a changed thigh profile.

The invention also provides a knee brace including an adaptable strapping mechanism for securing an upper portion of the brace to a user's thigh wherein the strapping mechanism automatically adjusts to underlying soft tissue motion during knee and leg movement, the strapping mechanism includes a topmost horizontal strap and a pair of lower crossing straps, the horizontal strap and the crossing straps alternately tighten and loosen during normal leg flexing and straightening so as to maintain a tight skeletal grip on the users leg while adapting to thigh muscle movement, and the individual straps of the strapping mechanism are independently adjustable to accommodate any leg size or shape.

In another aspect, the knee brace comprises a pair of lower rigid arms molded to fit on the anterior lower leg of a user, a cuff adapted to fit about a tibia of the user, a pair of upper rigid arms molded to fit on the anterior upper leg of a user, a hinge operably connecting each of said lower and upper rigid arms such that the hinge is located proximate a knee of the user, and a strapping mechanism straps adjustably connected between each of said upper arms wherein the strapping mechanism adapts to movement of the user.

In further aspect, the knee brace further comprises a tibial strap adapted to connect the lower arms to the lower leg of the user, and means for adjustably connecting the tibial strap snugly across the user's leg below the knee.

In still further aspect, the knee brace further comprises a protective flap secured to the upper rigid arms and adapted to extend across the thigh to cover the strapping mechanism.

In yet a further aspect, the knee brace comprise a pair of rigid arms having a hinge assembly near a midpoint thereof, a tibial cuff connected to a lower portion of the rigid arm and adapted to fit snugly about a lower leg of the user such that the hinge assembly is adjacent the user's knee, and a strapping mechanism for securing the knee brace an upper leg of the user, wherein the strapping mechanism conforms so movement off the user's thigh.

### BRIEF DESCRIPTION OF THE DRAWINGS

Various other objects, features and advantages of the present invention will become readily apparent by reading the following description in conjunction with the drawings, which are shown by way of example only, wherein:
Figure 1 is a front elevation of a knee the knee brace according to the present invention, showing
   the knee brace in a knee-extended position;
Figure 2 is a front elevation of a knee the knee brace according to the present invention, showing the knee brace in a knee-flexed position;
Figure 3 is detailed view of the upper strap arrangement for the knee brace according to the present invention;
Figure 4 is a perspective representation of the knee brace according to the present invention superimposed on a typical muscular arrangement of a human thigh;
Figure 5 is a graphical representation of a typical femoral profile of a human thigh; and
Figure 6, consisting of Figures 6A and 6B, represent the knee brace of the present invention responding to varus and valgus forces, respectively.

### DETAILED DESCRIPTION

The knee brace of the present invention provides numerous improvements over the prior art. For example, the brace contains at least one adjustable strap. The strap may be adjusted to ensure that the rigid thigh elements of the brace are correctly aligned with the long axis of the femur in the mid-medial and lateral lines. The adjustable strap also prevents hypertension in a changed thigh profile. The crossing straps can be adjusted so they fit a particular anterior thigh soft tissue profile and are equally tight in flexion and the rigid elements are not destabilized by soft tissue movement. Another advantage is grip, in terms of gripping the soft tissue of the user in to enable the brace to function effectively, and also to be able to provide skeletal grip in virtually all knee positions. Other features and advantages are described or will be apparent from the specification and claims.

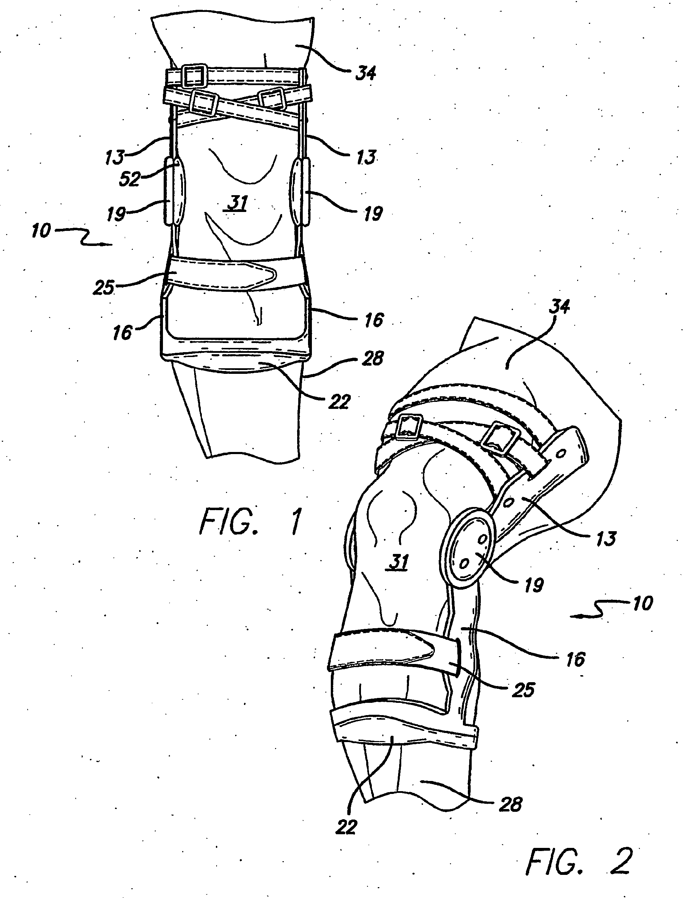

Referring now to the drawings in detail, wherein like reference characters refer to like elements, there is shown in Figures 1 and 2 a knee brace 10 according the present invention. Although this invention will be described by way of example and with reference to various preferred embodiments, it is to be understood that modifications or improvements may be made thereto without departing from the scope of the invention. Various changes and modifications to the presently preferred embodiments described herein will be apparent to those skilled in the art. Such changes and modifications may be made without departing from the present invention and without diminishing its attendant advantages. It is, therefore, intended that such changes and modifications are included within the present invention, which is limited only by the claims.

Referring to Figures 1 and 2, a knee brace 10 is shown having a biomechanical support comprising two substantially rigid upper 13 and lower 16 arms, which are joined together by a hinge assembly 19. Connected to the rigid lower arms is a lower or tibial cuff 22 that, together with adjustable tibial strap 25, secures the knee brace 10 to the users lower leg 28 below the knee 31. Preferably, the tibial cuff is made of a relatively strong yet lightweight fiber reinforced composite material. The hinge assembly 19 has a predetermined range of movement corresponding to the desired range of extension (Fig. 1) and flexion (Fig. 2) of the user's knee 31.

The upper arms 13 are secured to the users thigh (femur) 34 by a strapping mechanism 37. In this way the hinge assembly 19 is placed adjacent the axis of the user's knee joint, thereby allowing the knee brace 10 to substantially mimic the bending of the user's knee 31 as the user goes about his or her otherwise normal activities. The strapping mechanism 37 is designed to absorb the soft tissue profile change of the thigh, while the brace 10 still exerts a consistent skeletal force for proper support of the knee during normal knee 31 movements.

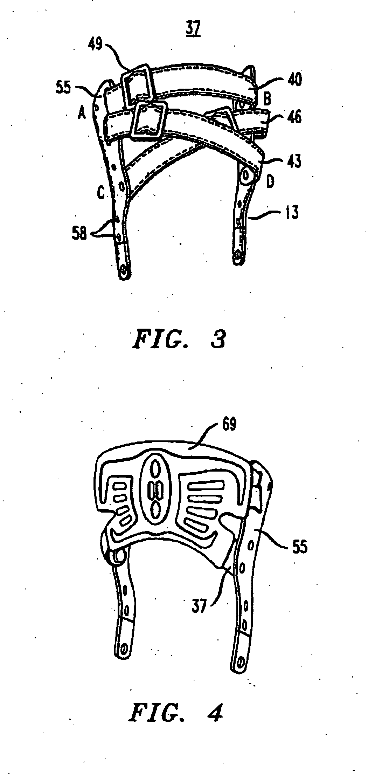

As represented by Figure 3, the strapping mechanism 37 may be attached to the upper arms 13 at four (4) basic attachment points, labeled A, B, C and D. The design of the strapping mechanism allows the relative position of points A, B, C and D to remain constant, whereas the area in between adapts to underlying soft tissue movement during normal knee motion. Points A, B, C and D are typically joined by three (3) straps .a first strap 40 operably connected between points A and B, a second strap 43 operably connected between points A and D, and a third strap 46 operably connected between points B and C. For each of the straps 40, 43, and 46, one end is fixed to one of the upper arms while an opposite end passes through a slot in the upper arm and is adjustably secured to itself by a buckle 49. As shown in Figure 3, the third strap 46 is pinned to one upper arm at point C, while the first and third straps 40 and 43 are pinned or secured to the other upper arm 13 at point B. It will be readily appreciate to those skilled in the art that the respective straps may be pinned at either end, so long as the other end is adjustable.

The straps 40, 43, and 46 adapt to the soft tissue movement during knee motion, which can be observed by comparing Figures 1 and 2. Figure 1 shows the brace 10 of the present invention with the knee 31 in extension or straight position, while Figure 2 shows the brace with the knee in flexion or bent position. When the leg is straight or fully extended, the first (horizontal) strap 40 is in a tightened state while the crossed second 43 and third straps 46 are in a less tightened (or relatively relaxed) state. In flexion, this condition is reversed: the first strap 40 is in a less tightened state while the crossed second and third straps 43 and 46 become increasingly tightened. Although these straps are described as becoming "relaxed", it will be understood by those skilled in the art that they still exert a force on the brace 10 to maintain the upper rigid arms 13 substantially parallel with the users thigh. This strapping mechanism 37 thus allows the knee brace 10 to spread in flexion and narrow in extension, just as the leg muscles normally do. This allows the quadriceps and hamstrings of the users upper leg to "fire" normally while the knee brace 10 maintains a predictable and constant force on the skeleton. Thus the users leg moves through the flexion-extension cycle the rigid thigh elements are held in the mid-medial and mid-lateral lines by a combination of strap tensions that absorb soft tissue movement. The quadriceps movement is multi-planar in so far as they rotate and move up and down with respect to the long axis of the thigh. In this manner, the straps transition between tension/maximum tension as the leg moves and the knee flexes/extends.

The knee brace of the present invention provides, for example, a three-point fixation centered at the knee that may be used to achieve the desired control over tibial movement with respect to the femur for proper knee bracing. The knee brace 10 provides a solid grip above the knee, at the knee and below the knee to provide the necessary control of the weakened knee joint. Because the strapping mechanism 37 for the knee brace 10 of the present invention adapts to the soft tissue movement, distal migration of the knee brace is eliminated. Hence the hinge 19 remains centered at the knee joint. Maintaining the hinge 19 in the proper relation with the knee 31 is desired to "push" the tibia into a slight valgus deformity, or less frequently, a slight varus deformity to counteract anterior translocation of the tibia with respect to the femur which can occur with ligament instability. A condylar pad 52 on the interior portion of each hinge 19 comfortably supports the knee joint in the lateral direction.

The adaptability of the strapping mechanism 37 for the knee brace of the present invention provides significant advantages over conventional bracing systems. In any population sample there will be a relatively small range of knee width and tibial size, but the range of thigh shape is much greater, and highly variable for any given knee and calf anatomical dimension. For any given individual the thigh shape to which a brace is to be applied will vary with degree of obesity or muscle definition, which tends to vary with exercise. It is therefore preferable to be able to have an adjustable soft or semi-rigid element linking the rigid elements on either side of the thigh. This component must allow for adjustment in several anatomical planes.

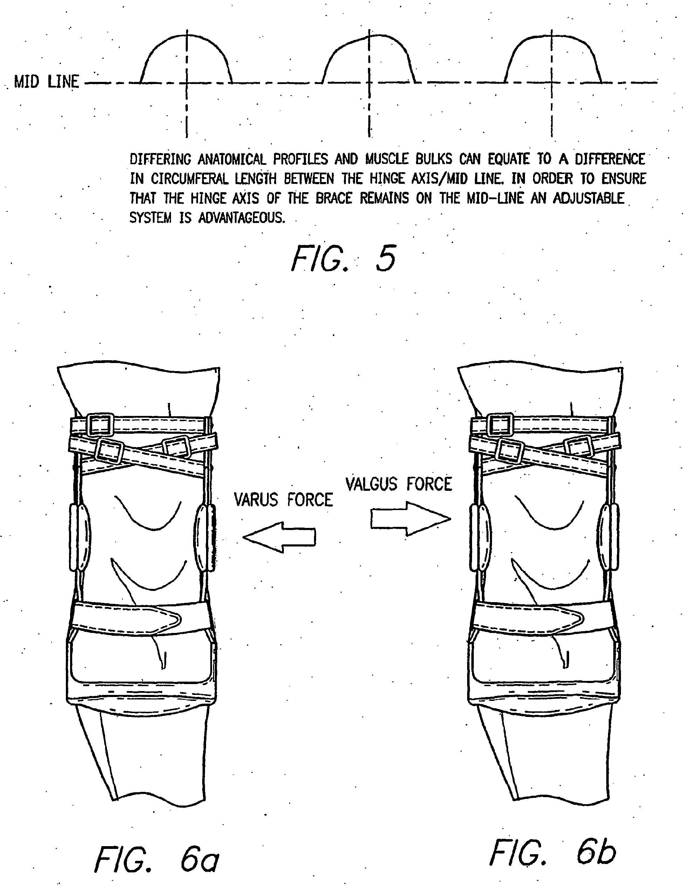

An objective of the horizontal strap 40 across the top of the strapping mechanism 37 is to hold the rigid thigh elements in line with the femur and prevent hyperextension (over-straightening) of the knee. The objective of the crossing second and third straps 43 and 46 is to grip the quadriceps and resist twisting of the knee brace 10, which is generally considered to be important to resist certain distorting forces applied to the knee (varus/valgus/rotation). See Figure 6.

An objective of the present invention is to provide an adjustable thigh section linking the rigid elements on either side of the thigh, and particularly one that is adjustable in several planes so that a rigid knee brace can be accurately fitted to, and intermittently adjusted for, any anterior thigh anatomical profile. This is beneficial because it allows accurate fitting to a wide range of profiles that can vary over time in any one individual. As shown in Figure *5*, thigh profile is a complicated shape that moves during a gait cycle in a variable way, determined by the relative size of the components of the quadriceps mechanism. The quadriceps is made up of component muscles that vary with activity level in any individual, so changes in overall quadriceps profile are frequent.

Accurate adjustment of the individual straps for the strapping mechanism 37 of the present knee brace 10 will not only provide an accurate and comfortable fit, but will ensure that the individual straps 40, 43, and 46 are appropriately tensioned to resist distorting forces or movements, and therefore provide a predictable skeletal force. Irrespective of the shape of the tibia! component, an anterior thigh component, adjustable in several dimensions, is beneficial in terms of fitting any individual thigh, because it is more adaptive to soft tissue profile. The result is that the knee brace 10 of the present invention is more positionally stable, and therefore exerts a more predictable and constant force on the underlying skeleton, which is the objective of knee bracing.

The instructions for fitting and adjusting the knee brace 10 of the present invention can be easily followed by a user, with or without prior instruction by any health care provider. First, the condylar pads 52 are positioned on each hinge 19 at the knee joint line on either side of the knee. The tibial cuff 22 is generally fitted to the lower leg 28 and cuff strap 25 tensioned for a firm but not tight fit. The rigid thigh flanges 55 extending from the upper arms 13 are thereby positioned on either side of the thigh 34. These should lie in the mid-medial and mid-lateral line parallel to the long axis of the femur. The individual straps of the strapping mechanism 37 should generally be in a loosened state. The horizontal first strap 40 extending between points A and B are adjusted so that in full extension the first strap 40 is tight across the thigh 34 and the rigid thigh flanges *55* lie parallel to the femur in the mid-medial and lateral lines, respectively. With the knee 31 flexed a predetermined amount, preferably at about 30 degrees, the crossing second and third straps 43 and 46 are individually adjusted to give a firm grip across the thigh 34. Preferably, the second and third straps are equally tensioned. When the knee is 31 then straightened, the crossing straps will be in the relaxed state and the horizontal strap will be in a tightened state.

The reason that it is preferable to adjust the crossing straps 43, 46 in 30 degrees of flexion is that this is the position where most typically instability is manifest clinically. It is desired to have these straps to be equally tensioned in this position so that the brace action is most accurate and predictable when the knee is most susceptible. If these straps are not adjustable to individual thigh profile then the brace is more susceptible to rotation (along with the quads) during the flexion/extension cycle. In other words, horizontal strap 40 is mainly responsible for stopping the rigid thigh elements moving out of position in the front/back anterior/posterior plane, while crossing straps 43,46 are mainly responsible for absorbing the rotation of the quads and therefore preventing rotation of the brace 10 around the long axis of the thigh during the cycle.

In a most preferred embodiment, it would be advantageous to be able to vary the position of points C and D. For a taller person with a longer thigh 34 it would be beneficial to lower the attachment points C and D, or move them closer to the knee 31. This may be achieved by moving the strap attachment point to one of several other predetermined positions 58 on the rigid thigh flanges 55.

Preferably the knee brace of the present invention provides various pads and liners between the rigid elements and the user's leg, as is conventional with braces in general. Also, a protective cuff or pad may be positioned over the strapping mechanism 37 to prevent unintentional adjustment of the buckles 49. Such a pad may be attached to the rigid thigh flanges in any conventional manner, such as, by way of example, with hook and loop fasteners (Velcro).

As can be seen from the foregoing, the invention provides a knee brace that includes functional elements that provides a number of different advantages.

Where in the foregoing description, reference has been made to specific components or integers of the invention having known equivalents then such equivalents are herein incorporated as if individually set forth. While specific embodiments of the invention have been shown in the drawings and described in detail, it will be appreciated by those skilled in the art that various modifications and alternatives would be developed in light of the overall teachings of the disclosure. Accordingly, the particular arrangements disclosed herein are meant to be illustrative only and not limiting as to the scope of the invention, which is to be given the full breadth of the appended claims and in any and all equivalents thereof.

## Claims

1. A knee brace (10) comprising:
a pair of lower rigid arms (16) molded to fit on the anterior lower leg (28) of a user;
a cuff (22) adapted to fit about a tibia of the user;
a pair of upper rigid arms (13) molded to fit on the anterior upper leg (34) of a user;
a hinge (19) operably connecting each of said lower and upper rigid arms (13, 16), such that the hinge (19) is located proximate a knee (31) of the user; and a strapping mechanism (37) adjustably connected between each of said upper rigid arms (13) wherein the strapping mechanism (37) adapts to movement of the user;
**characterized in that** said strapping mechanism (37) is comprised of a first strap (40) horizontally connected between an upper portion of each of the upper rigid arms (13), a second strap (43) diagonally connected between each of the upper rigid arms (13) and a third strap (46) diagonally connected between each of the upper rigid arms (13) such that the second and third straps (43, 46) cross over the upper leg (34) of the user below the first strap (40).

2. The knee brace (10) as in claim 1, further including a tibial strap (25) adapted to connect the lower arms (16) to the lower leg (28) of the user, and means for adjustably connecting the tibial strap (25) snugly across the user's leg below the knee (31).

3. The knee brace (10) as in claim 2, further including a protective flap secured to the upper rigid arms (13) and adapted to extend across the thigh (34) to cover the strapping a mechanism (37).

4. the knee brace (10) as in claim 1, wherein the first strap (40) is in a tightened state curing leg extension.

5. The knee brace (10) as in claim 1, wherein said second and third straps (43, 46) are in a relatively relaxed state during leg extension.

6. The knee brace (10) as in claim 1, wherein the first strap (40) is in a maximum tension state during leg extension, and said second and third straps (43, 46) are in a less tightened state during leg extension.

7. The knee brace (10) has in claim 1, wherein the first strap (40) is in a relatively relaxed state during leg flexion.

8. The knee brace (10) as in claim 1, wherein said second and third straps (43, 46) are in a tightened state during leg flexion.

9. The knee brace (10) as in claim 1, wherein said second and third straps (43, 46) are in a maximum tension state and the first strap (40) is in a less tightened state during leg flexion.

## Patentansprüche

1. Eine Kniestütze (10), aufweisend:
ein Paar von unteren steifen Armen (16), die geformt sind, um auf den anterioren Unterschenkel (28) eines Benutzers zu passen,
eine Manschette (22), die angepasst ist, um um ein Schienbein des Benutzers zu passen,
ein Paar von oberen steifen Armen (13), die geformt sind, um auf den anterioren Oberschenkel (34) eines Benutzers zu passen,
ein Gelenk (19), das jeden von den oberen und den unteren steifen Armen (13, 16) operativ miteinander verbindet, so dass das Gelenk (19) benachbart zu einem Knie (31) des Benutzers positioniert ist, und
einen Umschnallmechanismus (37), der zwischen jedem von den oberen steifen Armen (13) verstellbar angeschlossen ist, wobei sich der Umschnallmechanismus (37) der Bewegung des Benutzers anpasst,
**dadurch gekennzeichnet, dass** sich der Umschnallmechanismus (37) aus folgendem zusammensetzt: einem ersten Riemen (40), der horizontal zwischen einem oberen Abschnitt von jedem der oberen steifen Arme (13) verbunden ist, einem zweiten Riemen (43), der diagonal zwischen jedem von den oberen steifen Armen (13) verbunden ist, und einem dritten Riemen (46), der diagonal zwischen jedem der oberen steifen Arme (13) verbunden ist, so dass sich der zweite und der dritte Riemen (43, 46) über dem Oberschenkel (34) des Benutzers unterhalb des ersten Riemens (40) überkreuzen.

2. Die Kniestütze (10) gemäß Anspruch 1, ferner einen zum Verbinden der unteren Arme (16) mit dem Unterschenkel (28) des Benutzers angepassten Schienbeinriemen (25) und Mittel zum bequemen verstellbaren Verbinden des Schienbeinriemens (25) quer über das Bein des Benutzers unter dem Knie (31) aufweisend.

3. Die Kniestütze (10) gemäß Anspruch 2, ferner eine Schutzklappe aufweisend, die an den oberen steifen Armen (13) befestigt ist und angepasst ist, um sich über den Oberschenkel (34) zu erstrecken, um den Umschnallmechanismus (37) abzudecken.

4. Die Kniestütze (10) gemäß Anspruch 1, wobei der erste Riemen (40) während des Durchstreckens des Beins gestrafft ist.

5. Die Kniestütze (10) gemäß Anspruch 1, wobei der zweite und der dritte Riemen (43, 46) während des Durchstreckens des Beins in einem relativ entspannten Zustand sind.

6. Die Kniestütze (10) gemäß Anspruch 1, wobei sich der erste Riemen (40) während des Durchstreckens des Beins in einem maximalen Spannungszustand befindet und wobei sich der zweite und der dritte Riemen (43, 46) während des Durchstreckens des Beins in einem weniger gespannten Zustand befinden.

7. Die Kniestütze (10) gemäß Anspruch 1, wobei sich der erste Riemen (40) während des Beugens des Beins in einem relativ entspannten Zustand befindet.

8. Die Kniestütze (10) gemäß Anspruch 1, wobei sich der zweite und der dritte Riemen (43, 46) während des Beugens des Beins in einem gespannten Zustand befinden.

9. Die Kniestütze (10) gemäß Anspruch 1, wobei während des Beugens des Beins der zweite und der dritte Riemen (43, 46) sich in einem maximalen Spannungszustand befinden und der erste Riemen (40) sich in einem weniger gespannten Zustand befindet.

## Revendications

1. Une genouillère (10) comprenant :
Une paire de bras inférieurs rigides (16) moulés afin de s'adapter sur la partie inférieure antérieure de la jambe (28) d'un utilisateur ;
un manchon (22) adapté pour entourer le tibia d'un utilisateur ; une paire de bras supérieurs rigides (13) moulés pour s'adapter sur la partie supérieure antérieure de la jambe (34) d'un utilisateur ;
une articulation (19) reliant fonctionnellement chacun desdits bras inférieurs et supérieurs rigides (13, 16), de telle manière que l'articulation (19) soit située à proximité d'un genou (31) de l'utilisateur ; et
un mécanisme de la sangle (37) solidement fixé entre chacun desdits bras supérieurs rigides (13) dans lequel le mécanisme de la sangle (37) s'adapte au mouvement de l'utilisateur ;
dans lequel ledit mécanisme de la sangle (37) comprend une première sangle (40) reliée à l'horizontale entre une portion supérieure de chacun des bras supérieurs rigides (13), une seconde sangle (43) reliée en diagonale entre chacun des bras supérieurs rigides (13) et une troisième sangle (46) reliée en diagonale entre chacun des bras supérieurs rigides (13) de telle manière que la seconde et la troisième sangles (43, 46) se croisent sur la partie supérieure de la jambe (34) de l'utilisateur au-dessous de la première sangle (40).

2. La genouillère (10) selon la revendication 1, inclut également une sangle tibiale (25) adaptée pour relier les bras inférieurs (16) à la partie inférieure de la jambe (28) de l'utilisateur et des moyens pour fixer solidement la sangle tibiale (25) avec exactitude au travers de la jambe de l'utilisateur au-dessous du genou (31).

3. La genouillère (10) selon la revendication 2, inclut aussi un rabat de protection sécurisé aux bras supérieurs rigides (13) et adapté de manière à traverser au-dessus de la cuisse (34) pour couvrir le mécanisme de la sangle (37).

4. La genouillère (10) selon la revendication 1, dans laquelle la première sangle (40) est à l'état serré durant l'extension de la jambe.

5. La genouillère (10) selon la revendication 1, dans laquelle lesdites seconde et troisième sangles (43, 46) sont en état relativement relâché durant l'extension de la jambe.

6. La genouillère (10) selon la revendication 1, dans laquelle la première sangle (40) est dans un état maximum de tension durant l'extension de la jambe, et lesdites seconde et troisième sangles (43, 46) sont dans un état de tension moindre durant l'extension de la jambe.

7. La genouillère (10) selon la revendication 1, dans laquelle la première sangle (40) est dans un état relativement relâché durant la flexion de la jambe.

8. La genouillère (10) selon la revendication 1, dans laquelle lesdites seconde et troisième sangles (43, 46) sont dans un état de tension durant la flexion de la jambe.

9. La genouillère (10) selon la revendication 1, dans laquelle lesdites seconde et troisième sangles (42, 46) sont dans un état maximum de tension et la première sangle (40) est dans un état de tension moindre durant la flexion de la jambe.
